# FASCICULE DE BREVET EUROPEEN

(11) **EP 3 326 557 B1**
(45) Date de publication et mention de la délivrance du brevet: **26.06.2024**
(21) Numéro de dépôt: 17206342.2
(22) Date de dépôt: 17.02.2010
(51) Int. Cl.: A61B 17/80, A61B 17/00

(54) **ENSEMBLE SUR MESURE COMPRENANT AU MOINS DEUX PLAQUES D'OSTEOSYNTHESE QUI SUIVENT CHACUNE LA FUTURE ANATOMIE DU PATIENT**
PATIENTENSPEZIFISCHE ANORDNUNG MIT MINDESTENS ZWEI OSTEOSYNTHESEPLATTEN, DIE JEWEILS DER ZUKÜNFTIGEN ANATOMIE DES PATIENTEN FOLGEN
CUSTOMIZED ASSEMBLY COMPRISING AT LEAST TWO OSTEOSYNTHESIS PLATES THAT EACH FOLLOW THE FUTURE ANATOMY OF THE PATIENT

(30) Priorité: 17.02.2009 FR 0900722
(43) Date de publication de la demande: 30.05.2018
(62) Demande divisionnaire de: 14161014.7
(73) Titulaire: OBL (Société Anonyme), 92320 Chatillon (FR)
(72) Inventeur: LONGEPIED, Patrice, 92330 Sceaux (FR)
(74) Mandataire: EIP

(56) Documents cités:
- EP-A- 1 468 656
- FR-A- 2 626 460
- US-A1- 2002 128 654

## Description

La présente invention est relative à un procédé pour fabriquer un ensemble sur mesure d'au moins deux plaques d'ostéosynthèse, par exemple de types droites en I et/ou en L et/ou en croix et/ou en forme de patte-d'oie, elles-mêmes préparées sur mesure en fonction, d'une part, de l'opération à réaliser sur une mâchoire ou sur les deux mâchoires d'un patient et, d'autre part, de la future anatomie dudit patient.

On sait que, dans le cadre de la chirurgie plastique, en particulier orthognatique et traumatique, ou encore lors de certaines interventions chirurgicales, et notamment lors d'une intervention d'ostéosynthèse maxillomandibulaire - que cette dernière ne modifie pas l'occlusion ou qu'elle ne modifie pas les rapports occlusiaux ou encore qu'elle soit totale, en modifiant une arcade alvéolaire, ou segmentaire en n'intéressant qu'une partie de l'arcade - il est toujours nécessaire de recourir à la pose de plaques d'ostéosynthèse, c'est-à-dire de plaques de fixation vissées entre les parties osseuses qui se trouvent alors disjointes.

De telles plaques sont bien connues, en particulier du brevet français publié sous le n° 2.531.855 ou encore du brevet français publié sous le n° 2.725.124.

Ces plaques d'ostéosynthèse, qui sont le plus souvent réalisées en titane, sont tordues à la main par le chirurgien, avec des pinces, afin qu'elles s'adaptent parfaitement à la future anatomie du patient.

On sait par ailleurs réaliser, à partir des données d'un scanner ou d'une IRM, la simulation de l'ostéotomie grâce à laquelle on peut, au choix, avancer ou reculer la pièce osseuse du maxillaire et/ou de la mandibule jusqu'à, comme cela est souhaité, obtenir la disposition des dents du patient en regard les unes des autres.

Avant de procéder à une telle opération d'occlusion dentaire, on sait simuler par avance les résultats de l'opération sur le patient et l'on peut en conséquence préparer sur mesure des plaques d'ostéosynthèse de formes diverses qui devront être vissées aux endroits appropriés dans les éléments osseux d'une ou des deux mâchoires du patient, jusqu'à cicatrisation de l'os.

Cette préparation va jusqu'à donner elle aussi, ainsi qu'il a été dit en préliminaire, toutes les courbures voulues aux plaques d'ostéosynthèse, et ce afin d'adapter parfaitement ces dernières à la future anatomie du patient.

Le document EP1468656A décrit un dispositif d'ostéosynthèse ayant un corps fait d'un matériau biocompatible flexible, tel que du titane ou un alliage de titane. Le corps étant flexible et fourchu augmente l'adaptabilité du corps à fixer sur les fragments d'os. Le document FR2626460A décrit également une plaque d'os qui a une section flexible, allongée, pliable et en forme de tige pour le pontage d'une fracture osseuse. Lorsque la plaque osseuse est utilisée pour joindre un os fracturé, elle est courbée d'une manière choisie de manière à pouvoir se conformer substantiellement au contour de l'os sur le site de la fracture.

Cependant, de tels exemples de dispositifs d'ostéosynthèse flexibles peuvent reposer sur une manipulation manuelle du dispositif lors d'une opération pour adapter le dispositif à l'anatomie du patient.

Cela étant, et ce problème n'a pas encore été résolu, rien ne permet d'assurer que le chirurgien placera exactement aux bons endroits, sur la ou sur les deux mâchoires du patient, les plaques d'ostéosynthèse préparées sur mesure par avance.

La présente invention a pour but d'apporter une solution à ce problème en proposant un ensemble sur mesure d'au moins deux plaques d'ostéosynthèse, elles-mêmes préparées sur mesure. L'invention est définie dans les revendications annexées.

Dans des exemples, les plaques d'ostéosynthèse sont reliées entre elles par une tige. Dans certains exemples, laquelle tige comporte en outre deux proéminences qui émergent de ladite tige en direction de l'orifice nasal du patient en des positions qui correspondent aux deux extrémités de la base de l'orifice nasal du patient.

Dans certains exemples, les coins inférieurs de l'orifice nasal du patient ne pouvant varier en position, le fait pour le chirurgien de les utiliser comme références naturelles pour loger les proéminences de la structure d'ensemble selon l'invention et, à cette suite, pour placer ladite structure d'ensemble soit sur la mâchoire inférieure, soit sur la mâchoire supérieure du patient concernée par l'opération, garantit que toutes les plaques d'ostéosynthèse de ladite structure occuperont sans aucune exception les emplacements qu'il a par avance été prévu qu'elles tiennent.

Avantageusement, les deux proéminences ont chacune la forme d'un téton, d'un doigt, d'un ergot ou d'un éperon. Les deux repères en saillie ainsi prévus par construction sur la tige de la structure d'ensemble selon l'invention étant des proéminences qui viennent se nicher dans les coins inférieurs de l'orifice nasal du patient, la pose par le chirurgien de ladite structure d'ensemble, avec la plus grande précision qui soit, en est facilité.

Toujours avantageusement, la tige de la structure d'ensemble selon l'invention respecte elle-aussi parfaitement l'anatomie du patient.

Dans une autre forme préférée de réalisation, la structure d'ensemble selon l'invention se caractérise par le fait que, à proximité de chacune de ses plaques, la tige de ladite structure présente des zones de moindre résistance destinées à faciliter la coupe par le chirurgien de toutes les parties de liaison entre les différentes plaques d' ostéosynthèse de ladite structure.

Après son placement en position idéale, par le chirurgien, de la structure d'ensemble selon l'invention, puis fixation de toutes les plaques d' ostéosynthèse par vissage dans l'os de la mâchoire supérieure ou de la mâchoire inférieure du patient, il est à l'évidence souhaitable de faire immédiatement disparaître toutes les parties de liaison entre lesdites plaques, y compris la partie qui présente les proéminences ayant permis la mise précise en position idéale de la structure d'ensemble sur la mâchoire du patient. Les zones de moindre résistance prévues à proximité immédiate des plaques ne feront que simplifier la tâche du chirurgien tout au long de cette opération d'élimination des parties de la structure d'ensemble selon l'invention devenues inutiles.

La structure d'ensemble selon l'invention est avantageusement fabriquée d'une seule pièce et, de préférence, elle est réalisée en titane.

Dan un exemple, a également pour objet un procédé de mise en place d'au moins deux plaques d'ostéosynthèse sur le maxillaire et/ou sur la mandibule d'un patient, lors d'une opération d'occlusion dentaire, ledit procédé étant caractérisé en ce que, en préliminaire de ladite opération, l'on simule par avance les résultats de l'opération sur le patient, l'on détermine la position et la forme des plaques d'ostéosynthèse en fonction de la future anatomie du patient, l'on réalise sur mesure un ensemble d'au moins deux plaques d'ostéosynthèse, par exemple de types droites en I et/ou en L et/ou en croix et/ou en forme de patte-d'oie, elles-mêmes réalisées sur mesure en fonction de l'opération à réaliser et de la future anatomie du patient, ledit ensemble comprenant une tige qui relie entre elles les plaques d'ostéosynthèse et qui comporte en outre deux proéminences correspondant aux coins inférieurs de l'orifice nasal du patient, et en ce que, après l'opération, le chirurgien place idéalement ledit ensemble sur mesure en disposant ses deux proéminences dans les coins inférieurs de l'orifice nasal du patient, puis il fixe toutes les plaques d'ostéosynthèse à l'aide de vis pénétrant les éléments osseux de la mâchoire supérieure ou de la mâchoire inférieure concernée, puis il coupe à proximité de chacune des plaques d'ostéosynthèse dudit ensemble toutes les parties de liaison formées par la tige entre lesdites plaques.

L'invention sera mieux comprise à la lecture de la description qui va suivre, et en référence aux dessins annexés qui illustrent de manière non limitative un mode de réalisation de ladite invention, et sur lesquels :
- les Figures 1 et 2 représentent, vu de face et respectivement vu de côté, un exemple de réalisation de la structure d'ensemble sur mesure de plaques d'ostéosynthèse selon l'invention, ladite structure d'ensemble comportant quatre plaques en L dans un tel exemple.
- la Figure 3 illustre en vue de face le placement en position idéale sur la mâchoire supérieure d'un patient de l'ensemble sur mesure des Figures 1 et 2.
- la Figure 4 illustre en vue de profil le même placement en position idéale de l'ensemble sur mesure des Figures 1 et 2 sur la mâchoire supérieure du patient.

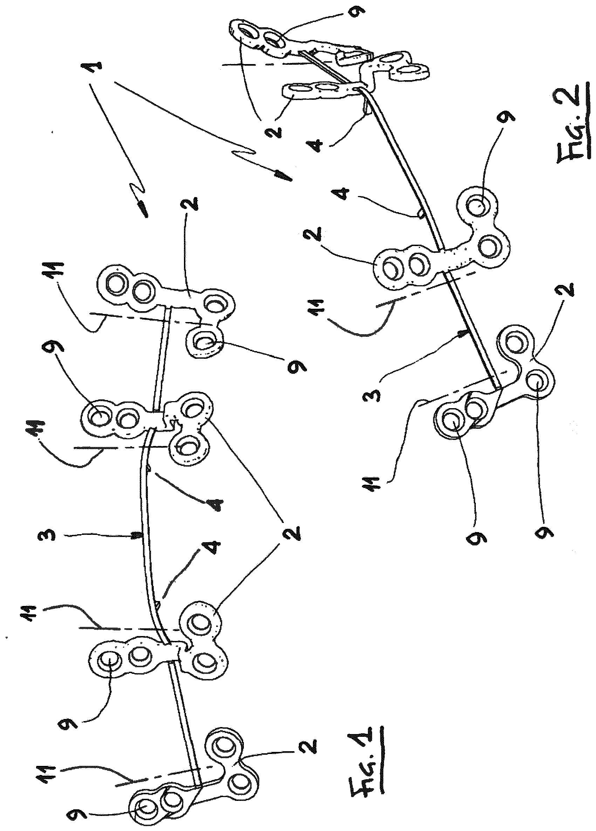

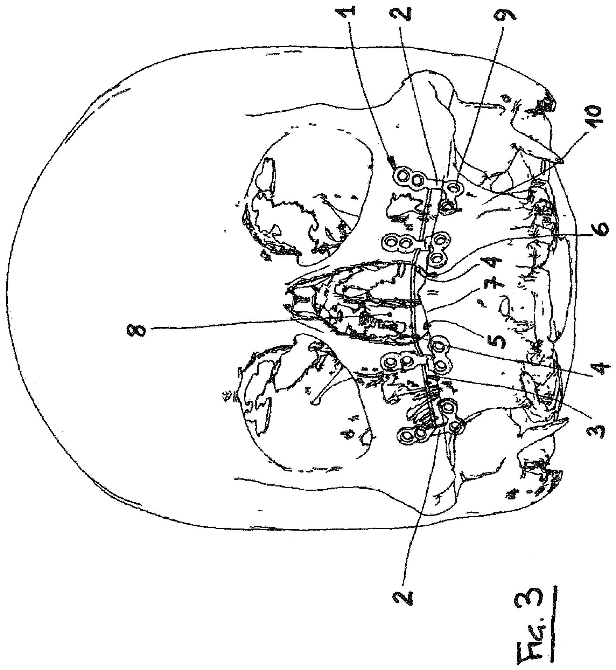

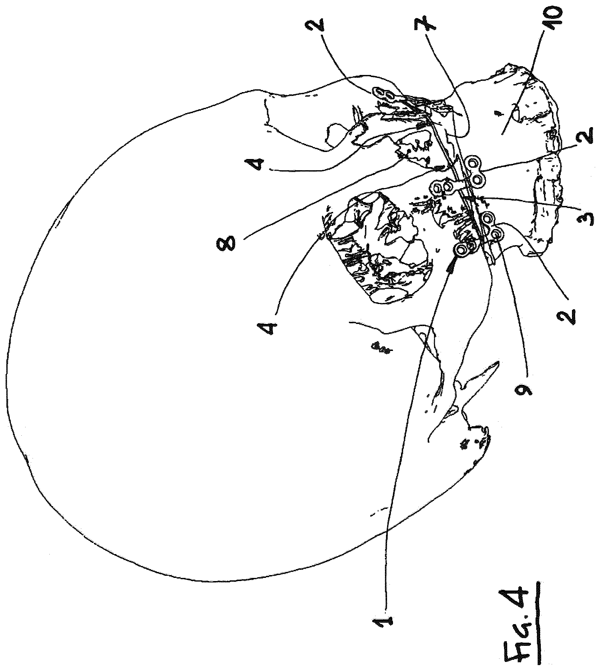

Dans l'exemple choisi, représenté sur les Figures 1 à 4, l'opération d'occlusion dentaire réalisée porte exclusivement sur la mâchoire supérieure, encore appelée maxillaire.

En variante, il est toutefois bien clair que l'invention s'applique également à toute réparation de la mâchoire inférieure, encore appelée mandibule, que cette réparation soit réalisée seule ou concomitamment avec celle de la mâchoire supérieure.

Dans cette variante, seules seront modifiées la forme de la tige de liaison ainsi que les formes des plaques d'ostéosynthèse qui, pour la réparation du maxillaire, sont le plus souvent du type en L et/ou en croix et/ou en forme de patte-d'oie.

En effet, pour la réparation de la mandibule, il leur est généralement préféré des plaques d'ostéosynthèse en forme de I, c'est-à-dire des plaques droites.

Connaissant la future anatomie du patient à partir des données d'un scanner ou d'une IRM, on réalise conformément à l'invention, en préliminaire de l'opération convenue, un ensemble 1 d'au moins deux plaques d'ostéosynthèse, quatre en L dans l'exemple représenté sur les Figures 1 à 4, qui est formé :
- des plaques d'ostéosynthèse 2 à proprement parler, lesquelles, en fonction précisément de la future anatomie du patient, et donc des positions qu'elles devront occuper sur sa mâchoire à réparer, sont mises chacune à la forme appropriée, c'est-à-dire sur mesure, avec ici et là les courbures qui leur garantiront d'épouser parfaitement la future anatomie désirée,
- une tige 3 qui relie entre elles toutes les plaques 2, ladite tige comportant en outre deux proéminences 4 correspondant très exactement aux deux extrémités, respectivement 5 et 6, de la base 7 de l'orifice nasal 8 du patient.

Chaque plaque d'ostéosynthèse 2 est, de façon connue, percée de plusieurs trous traversants 9, ici quatre trous pour chaque plaque en forme de L, lesquels trous sont destinés à recevoir les vis qui, après la mise en place parfaite de l'ensemble 1 (encore appelé structure d'ensemble) sur le maxillaire 10 du patient, assureront la fixation dudit ensemble sur ledit maxillaire en étant vissées, par le chirurgien dans les parties osseuses qui, disjointes après l'opération, doivent être réunies en étant placées les unes contre les autres.

Les deux proéminences 4 sont par exemple avantageusement en forme de tétons ou de doigts, ou d'ergots, ou d'éperons, lesquels émergent de la tige 3 en direction de l'orifice nasal 8 du patient. Toujours par construction, les positions sur la tige et les formes de ces proéminences 4 ont été déterminées à partir des données connues des scanners ou IRM.

De préférence, la tige 3 respecte elle-aussi parfaitement l'anatomie de l'os maxillaire ou, selon, celle de l'os mandibulaire du patient ; ses parties courbes et rectilignes sont donc connues à partir des données précitées et elles sont ainsi reproduites à l'identique sur la tige lors de la construction de la structure d'ensemble.

Après avoir effectué l'opération désirée, le chirurgien place les unes contre les autres les parties osseuses disjointes, puis il met en place la structure 1 en disposant les deux proéminences 4 dans les coins inférieurs 5 et 6 de l'orifice nasal 8 du patient, c'est-à-dire en les logeant aux deux extrémités de la base 7 dudit orifice nasal.

La structure 1 occupe idéalement sa place, déterminée à l'avance, par rapport au maxillaire du patient et le chirurgien n'a plus alors qu'à procéder à la fixation de ladite structure sur le maxillaire à l'aide des vis traversant les trous 9 des plaques d'ostéosynthèse 2 en L.

Les parties osseuses disjointes étant ainsi réunies fixement, et devant l'être jusqu'à la cicatrisation de l'os, le chirurgien n'a plus qu'à éliminer de la structure 1 toutes les parties de celle-ci devenues inutiles, c'est-à-dire les parties de liaison entre les différentes plaques d'ostéosynthèse 2, seules lesdites plaques demeurant donc dans la bouche du patient en fin d'opération.

Pour cela, le chirurgien coupe la tige 3 à proximité immédiate de chacune des plaques 2, y compris la partie de la tige qui comprend les deux proéminences 4.

Afin de faciliter ce travail de coupe de la tige en vue de son élimination, on prévoit par construction que ladite tige présente au voisinage de chacune des différentes plaques 2 des zones de moindre résistance illustrées par les traits mixtes 11, ces derniers, pour une meilleure clarté des dessins, n'ayant été schématisés qu'en quelques occasions.

L'ensemble sur mesure 1 selon l'invention - plaques d'ostéosynthèse 2 et tige 3 - est avantageusement fabriqué en titane, matériau biocompatible d'usage maintenant courant en chirurgie, et ledit ensemble est de préférence réalisé d'une seule pièce.

Comme il va de soi, l'invention ne se limite pas aux seules spécifications techniques données ci-dessus à titre d'exemples ; elle embrasse, au contraire, toutes les variantes possibles de réalisation, en particulier celle de la construction d'une autre structure 1 adaptée spécifiquement à la réparation d'une mandibule, autre structure pour laquelle, d'une part, la tige 3, au lieu d'être sensiblement rectiligne, aura davantage la forme d'un oméga et, d'autre part, des plaques 2 en forme de I viendront selon la tradition se substituer aux plaques choisies dans le présent exemple en forme de L, mais qui pourraient aussi bien être en croix ou en forme de patte-d'oie.

## Revendications

1. Procédé pour fabriquer un ensemble sur mesure (1) comprenant au moins deux plaques d'ostéosynthèse (2), le procédé comprenant les étapes suivantes :
obtenir une simulation d'une opération sur une mâchoire d'un patient, dans laquelle opération, une première partie de la mâchoire est disjointe d'une seconde partie de la mâchoire, et où la simulation de l'opération comprend une future anatomie envisagée du patient, basée sur l'opération simulée ;
concevoir un ensemble sur mesure (1) comprenant au moins deux plaques d'ostéosynthèse (2) qui suivent chacune la future anatomie du patient,
la conception de l'ensemble sur mesure (1) comprenant concevoir l'ensemble sur mesure (1) pour qu'il comprenne une tige (3) pour raccorder les au moins deux plaques d'ostéosynthèses (2) entre elles ; et
fabriquer l'ensemble sur mesure (1).

2. Procédé selon la revendication 1, dans lequel la conception comprend la conception de la tige (3) de sorte que la tige (3) comprend deux saillies (4) dans des positions qui correspondent aux deux extrémités (5, 6) d'une base (7) d'un orifice nasal (8) du patient.

3. Procédé selon la revendication 1 ou 2, dans lequel la conception comprend la conception de la tige (3) de sorte que la tige (3) suit la future anatomie du patient.

4. Procédé selon l'une quelconque des revendications précédentes, dans lequel la fabrication comprend la fabrication de l'ensemble sur mesure (1) d'un seul tenant.

5. Procédé selon l'une quelconque des revendications précédentes, dans lequel la fabrication comprend la fabrication de l'ensemble sur mesure (1) avec du titane.

6. Procédé selon l'une quelconque des revendications précédentes, dans lequel la mâchoire du patient comprend un maxillaire supérieur (10) ou un maxillaire inférieur du patient.

7. Procédé selon l'une quelconque des revendications précédentes, comprenant la conception des au moins deux plaques d'ostéosynthèse (2) pour qu'elles comprennent chacune des trous débouchants afin de loger des vis pour fixer les au moins deux plaques d'ostéosynthèse (2) à la mâchoire du patient.

8. Procédé selon la revendication 7, comprenant la conception des au moins deux plaques d'ostéosynthèse (2) pour qu'elles comprennent chacune un trou débouchant (9) correspondant à la première partie de la mâchoire et un trou débouchant correspondant à la seconde partie de la mâchoire.

9. Procédé selon l'une quelconque des revendications précédentes, comprenant la conception de la tige (3) pour qu'elle comprenne une ou plusieurs zones de résistance réduite (11) destinées à être utilisées pour retirer une partie de la tige (3).

## Patentansprüche

1. Verfahren zum Herstellen einer maßgefertigten Anordnung (1), umfassend mindestens zwei Osteosyntheseplatten (2), das Verfahren umfassend die folgenden Schritte:
Erhalten einer Simulation einer Operation an einem Kiefer eines Patienten, wobei bei der Operation ein erster Teil des Kiefers von einem zweiten Teil des Kiefers getrennt wird, und wobei die Simulation der Operation eine geplante zukünftige Anatomie des Patienten umfasst, basierend auf der simulierten Operation;
Entwerfen einer maßgefertigten Anordnung (1), umfassend mindestens zwei Osteosyntheseplatten (2), die jeweils der zukünftigen Anatomie des Patienten folgen,
das Entwerfen der maßgefertigten Anordnung (1) umfassend das Entwerfen der maßgefertigten Anordnung (1) damit sie einen Stift (3) zum Verbinden der mindestens zwei Osteosyntheseplatten (2) miteinander umfasst; und
Herstellen der maßgefertigten Anordnung(1).

2. Verfahren nach Anspruch 1, wobei das Entwerfen das Entwerfen des Stifts (3) so umfasst, dass der Stift (3) zwei Vorsprünge (4) an Positionen umfasst, die den zwei Enden (5, 6) einer Basis (7) einer Nasenöffnung (8) des Patienten entsprechen.

3. Verfahren nach Anspruch 1 oder 2, wobei das Entwerfen das Entwerfen des Stifts (3) so umfasst, dass der Stift (3) der zukünftigen Anatomie des Patienten folgt.

4. Verfahren nach einem der vorstehenden Ansprüche, wobei das Herstellen das Herstellen der maßgefertigten Anordnung (1) in einem Stück umfasst.

5. Verfahren nach einem der vorstehenden Ansprüche, wobei das Herstellen das Herstellen der maßgefertigten Anordnung (1) mit Titan umfasst.

6. Verfahren nach einem der vorstehenden Ansprüche, wobei der Kiefer des Patienten einen Oberkiefer (10) oder einen Unterkiefer des Patienten umfasst.

7. Verfahren nach einem der vorstehenden Ansprüche, umfassend das Entwerfen der mindestens zwei Osteosyntheseplatten (2) damit sie jeweils Durchgangslöcher umfassen, um Schrauben zum Befestigen der mindestens zwei Osteosyntheseplatten (2) an dem Kiefer des Patienten aufzunehmen.

8. Verfahren nach Anspruch 7, umfassend das Entwerfen der mindestens zwei Osteosyntheseplatten (2) damit sie jeweils ein Durchgangsloch (9), das dem ersten Kieferteil entspricht, und ein Durchgangsloch, das dem zweiten Kieferteil entspricht, umfassen.

9. Verfahren nach einem der vorstehenden Ansprüche, umfassend das Entwerfen des Stifts (3) damit er einen oder mehrere Bereiche mit verringertem Widerstand (11) umfasst, die dafür bestimmt sind, zum Entfernen eines Teils des Stifts (3) verwendet zu werden.

## Claims

1. A method of manufacturing a customized assembly (1) comprising at least two osteosynthesis plates (2), the method comprising the following steps:
obtaining a simulation of an operation on a jaw of a patient, in which operation a first part of the jaw is disjoined from a second part of the jaw, and wherein the simulation of the operation comprises an envisaged future anatomy of the patient, based on the simulated operation;
designing a customized assembly (1) comprising at least two osteosynthesis plates (2), each of which follows the future anatomy of the patient,
designing the customized assembly (1) comprising designing the customized assembly (1) to comprise a rod (3) for connecting the at least two osteosynthesis plates (2) together; and
manufacturing the customized assembly (1).

2. The method according to claim 1, wherein the design comprises designing the rod (3) so that the rod (3) comprises two projections (4) in positions which correspond to the two ends (5, 6) of a base (7) of a nasal orifice (8) of the patient.

3. The method according to claim 1 or 2, wherein the design comprises designing the rod (3) so that the rod (3) follows the future anatomy of the patient.

4. The method according to any one of the preceding claims, wherein the manufacture comprises manufacturing the customized assembly (1) in a single piece.

5. The method according to any one of the preceding claims, wherein the manufacture comprises manufacturing the customized assembly (1) with titanium.

6. The method according to any of the preceding claims, wherein the patient's jaw comprises an upper maxilla (10) or a lower maxilla of the patient.

7. The method according to any one of the preceding claims, comprising designing the at least two osteosynthesis plates (2) so that they each comprise through-holes for accommodating screws for securing the at least two osteosynthesis plates (2) to the jaw of the patient.

8. The method according to claim 7, comprising designing the at least two osteosynthesis plates (2) so that they each comprise a through hole (9) corresponding to the first part of the jaw and a through hole corresponding to the second part of the jaw.

9. The method according to any of the preceding claims, comprising designing the rod (3) to comprise one or more zones of reduced resistance (11) for use in removing a part of the rod (3).
